# EUROPEAN PATENT SPECIFICATION

(11) **EP 4 069 901 B1**
(45) Date of publication and mention of the grant of the patent: **06.08.2025**
(21) Application number: 20811649.1
(22) Date of filing: 27.11.2020
(51) Int. Cl.: D04H 1/728, A61F 2/00, D06M 10/10

(54) **ADAPTIVE CHEMICAL POST-PROCESSING OF NONWOVENS FOR CARDIOVASCULAR APPLICATIONS**
ADAPTIVE CHEMISCHE NACHBEHANDLUNG VON VLIESSTOFFEN FÜR KARDIOVASKULÄRE ANWENDUNGEN
TRAITEMENT CHIMIQUE ULTÉRIEUR ADAPTATIF DES NON-TISSÉS POUR APPLICATIONS CARDIOVASCULAIRES

(30) Priority: 03.12.2019 DE 102019132800; 31.03.2020 EP 20167129
(43) Date of publication of application: 12.10.2022
(73) Proprietor: Cortronik GmbH, 18119 Rostock-Warnemünde (DE)
(72) Inventor: OSCHATZ, Stefan, 18057 Rostock (DE); ILLNER, Sabine, 18059 Rostock (DE); ORTELT, Jonathan, 18109 Rostock (DE); ARBEITER, Daniela, 18069 Rostock (DE); TESKE, Michael, 18109 Rostock (DE); MUELLER, Heinz, 18055 Rostock (DE); GRABOW, Niels, 18055 Rostock (DE); MOMMA, Carsten, 18057 Rostock (DE); SCHMITZ, Klaus-Peter, 18119 Rostock (DE)
(74) Representative: Biotronik Corporate Services SE
(86) International application number: PCT/EP2020/083711
(87) International publication number: WO 2021/110559

(56) References cited:
- EP-A1- 3 366 362

## Description

The present invention relates to methods for post-processing electrospun nonwoven materials to control the mechanical properties and surface finish, and to nonwoven materials formed from electrospun fibres.

Electrospun nanofibre nonwovens formed from medically approved permanent or degradable polymers are known, for example as patch structures, graft systems or sheathings of supporting structures for innovative implant modification in the field of medical technology. The possibility of generating nanofibre nonwovens from a variety of polymers by electrospinning processes combines the mechanical robustness of randomly interwoven fibres with the chemical-biological properties of the underlying plastics. In particular, the possibility of processing in controlled processes is an immense advantage over the use of biogenic materials, such as pericardium, for example in the production of heart valve replacement materials. In addition, active substances can be incorporated into the synthetically produced nonwoven structures as well as into film-like coatings, thus generating drug delivery systems. The coating of vascular supports (stents) with nonwoven structures to create sealing stent grafts is well established; commercially available examples are the following: Papyrus (Biotronik), Jostent Graft-Master (Abbott) and Bioweb (Zeus). Electrospun fibers are described in EP 3 366 362 A1.

Furthermore, it has already been described that materials for the prevention of paravalvular leakages are producible by means of a combination and subsequent mechanical interlacing of a number of special fibres.

US 2015/0297372 A1 (Abbott Cardiovascular Systems Inc), US 2013/0150943 A1 (Elixir Medical Corp), EP 2 796 112 A1 (Elixir Medical Corp), EP 2 104 521 B1 (Medtronic Vascular Inc) and WO 2005/034806 A1 (Scimed Life Systems Inc) comprise degradable stents consisting of a polymer or metal main body, which may also have a degradable coating. However, the stent systems do not include a cover modified by post-processing. The patent specifications EP 2 596 765 B1 (Kyoto Medical Planning Co., Ltd) and EP 1 919 532 B1 (Boston Scientific Corp) describe covered stent systems, in which only the cover or the stent scaffold is degradable. A degradable stent scaffold with a permanent cover is also described by WO 2004/016192 A1 (Scimed Life Systems Inc). In this case, however, the cover is located within the stent scaffold, the function of which is mainly to prevent the implant from dislocating in the vessel. A system in which the cover and stent scaffold are degradable is described by WO 2010/117538 A1 (Medtronic Vascular Inc). In addition, EP 2 380 526 A2 describes an implant and a method for its production, which implant comprises a covered stent, which may also contain a degradable cover.

At present, a range of products are available that contain nanofibre nonwovens that can be obtained from biocompatible polymers by electrospinning processes. However, without chemical post-treatment, the resulting nonwoven structures are limited to the physico-chemical properties inherent to the polymers used. For example, polyurethane nonwovens show a sudden drop in tensile strength in an aqueous environment, which is due to a splitting of intramolecular hydrogen bonds. Furthermore, currently the fibre thickness (fibre diameter) and the physical bonds between the electrospun fibres are only dependent on the underlying spinning parameters.

The generation of high-performance materials based on electrospun nanofibre nonwovens for implant development is usually based on the use of hybrid materials. These consist of nonwovens of different interwoven fibres formed from different polymers in order to achieve a combination of material properties, including an increase in the tensile strength of the material. Other possibilities include, for example, the spinning of mixtures of different polymers from a single solution, so-called polymer blends, or the application of an additional polymer layer by dipping or spraying processes, although this is accompanied by a loss of the characteristic nonwoven surface morphology.

A post-process chemical modification to adjust the material properties, in particular while retaining the typical nonwoven fibre structure for biomedical applications, is not yet known.

Therefore, the object of the present invention is to provide a medical implant comprising a nonwoven material according to claim 1. Improved nonwoven materials for biomedical applications, in particular while preserving the typical nonwoven fibre structure, are described.

This object is achieved by the fibre nonwoven materials proposed herein and their use in medical devices. In particular, a solution can be seen in the post-process adjustment of the physico-chemical properties as well as the tissue-implant reaction of polymer-based implants to specifically defined parameters for the generation of high-performance materials starting from electrospun nanofibre nonwovens. In particular, the invention presents a possibility for carrying out spatially resolved material modifications while preserving the nonwoven fibre structure, since this filigree fibre structure can be made mainly responsible for the suitability as implant material, such as atrioventricular valve replacement material or sheathing material.

Accordingly, a non-woven material formed from electrospun fibres, in particular nanofibres, is proposed which has a surface modification by which the fibres may be crosslinked with each other. In particular, the electrospun fibres are crosslinked. In one embodiment, the surface modification is present in the form of chemical reactive groups. In one embodiment, the fibre material is selected from the group comprising or consisting of polyurethanes, polyimides, polyamides, polyesters and polytetrafluoroethylenes.

The chemical reactive groups may be selected here from the group comprising or consisting of diisocyanates, alcohols, epoxides, imides, amides, imines, amines, especially allylamines, diacrylates, disiloxanes and disilazanes.

According to the invention, the introduction of surface modification allows the mechanical properties, in particular tensile strength and plastic elongation range, to be adapted advantageously by subsequently blocking the mobility of the polymer chains via (photo)chemical or thermal crosslinking, which affects surface polarity, in particular with regard to biocompatibility, cell growth and endothelialisation. When using biodegradable materials, which optionally carry an active substance, the speed at which degradation or active substance release processes occur can also be adjusted, which can be achieved by the post-process creation of a diffusion barrier.

The above-mentioned modifications can be carried out with easily realisable process steps, while also preserving the typical fibre structure of the electrospun nonwovens, as these are mainly responsible for the mechanical suitability of nanofibre nonwovens in the cardiovascular area. Therefore, the present invention addresses the possibility of modifying electrospun nanofibre nonwovens while preserving the fibre structure post-process, i.e. after the actual spinning process, by treatment with heat or UV radiation, or by applying thin or ultra-thin layers by vapour deposition or PECVD technologies with respect to their physico-chemical properties in such a way that they meet the requirements of the particular site of use as an implant. This means that nonwoven materials tailored to the site of use can be made accessible in just a few processing steps.

The biocompatible nonwoven materials created in this way open up new fields of application in the development of implants in terms of their material properties. In particular, the fields of application constituted by stents, TAVI procedures, and coatings for implants, for example for pacemaker and implantable defibrillator electrodes, are addressed.

In one embodiment, a nonwoven material formed from electrospun fibres, in particular nanofibres, is proposed, having a surface modification by which the fibres can be crosslinked with one another, characterised in that the fibre material is a polyimide and the surface modification is in the form of diisocyanates or amines, in particular diamines.

Such an embodiment has the advantage that delamination problems can be eliminated. This is because one technical problem is the tendency of some polymers, such as polyimide, to delaminate too much after they have been spun into nonwovens. More precisely, there is insufficient physical crosslinking (bonding) between two or more fibres (unbonded fibre fabrics). This can be counteracted by the subsequent application of a fixation layer by (plasma-activated) vaporisation or spraying processes. According to the invention, this layer consists of reactive, volatile molecules, for example 1,6-diisocyanatohexane (HMDI) or 1,2-ethylene diamine, which precipitates from a saturated gas phase on the surface of the nonwoven and fixes the polymer fibres at the contact points, thus creating a dimensionally stable network.

A further aspect of the present invention is a method for producing a crosslinked electrospun nonwoven material comprising the steps of:
- providing a fibre material,
- electrospinning the fibre material in the form of fibres into a nonwoven material,
- introducing a surface modification to the fibres either by modifying the fibre material before the electrospinning or by modifying the fibre surface after the electrospinning,
- crosslinking the fibres to form a crosslinked nonwoven material.

In one embodiment, it is provided that the introduction of a surface modification is carried out by adding a reactive chemical substance to the fibre material before the electrospinning. In one embodiment, the surface modification is achieved by adding diisocyanates, alcohols, epoxides, imides, amides, imines, amines, in particular allylamines, diacrylates, disiloxanes and disilazanes.

In one embodiment, the active chemical groups are added to the polymer already before the spinning process. In subsequent post-processing treatment, among other things, the loss of tensile strength in the medium can be compensated for by increasing the tensile strength overall.

By introducing reactive chemical substances during the spinning process, individual nonwoven layers can also be irreversibly bonded together by chemical bonds during post-processing. Therefore, nonwoven-like composite materials can be created with a layered structure (sandwich structure), while preserving the fibre structure. Depending on the choice of the polymers used, these composites exhibit novel physico-chemical properties that differ greatly from the material properties of the individual components. This technology represents an alternative to established contact welding or bonding methods, for example to embed the struts of a stent scaffold in the nonwoven.

By introducing reactive chemical substances, such as diisocyanates or diacrylates, into, for example, thermoplastic silicone polycarbonate-urethanes (TSPCUs) prior to the actual spinning process, the system can be additionally crosslinked at the molecular level through activation processes, such as thermal treatment (annealing) or treatment with UV radiation, while maintaining the same level of processing. This can be used to adjust the material hardness as well as to generate joins.

In a further embodiment of the method proposed herein, the introduction of a surface modification is carried out by applying a reactive chemical substance to the fibre material after electrospinning. This can be achieved, for example, by vapour deposition with reactive components using highly volatile reactants. This enables additional chemical as well as physical connections between different fibres of the polymer nonwoven at the fibre surface. Among other things, a mutual displacement of the individual fibres can be blocked. As the nonwoven fibres still have sufficient mobility after the treatment, the pronounced extensibility of the material can be extended by an increased tensile strength.

In a preferred embodiment, the introduction of a reactive chemical substance to the fibre material is carried out at elevated temperature, especially in the range of 50 to 90°C. In this temperature range, the morphology of the nonwoven remains unchanged, while it is possible to transfer a high concentration of the reactive chemical substance to the nonwoven fibres, which allows a strong crosslinking of the nonwoven fibres. In another embodiment of this process step, the introduction is performed at an elevated temperature in the presence of water, especially in the presence of (water) vapour.

Alternatively, there is the possibility of creating a barrier layer as a diffusion barrier by plasma-chemical application of ultra-thin polymer layers *via* plasma polymerisation (PECVD, *plasma-enhanced chemical vapour deposition*), which, for example, prevents hydration of urethane groups. The advantage of this process lies in the low thermal load on the nanofibre nonwoven structures, as the reaction energy is supplied by a plasma instead of by temperature, and the homogeneous layer formation in the nanometre range. Depending on the choice of the precursor monomers in the gas space of the reaction chamber, this layer can be biostable or biodegradable and thus may have an effect on the degradation behaviour of biodegradable nonwovens. In addition, this layer can modulate the release of active substances incorporated in the nonwoven and may significantly reduce a burst release.

The additional chemical post-processing advantageously makes it possible to individually adjust and increase the morphology of the obtained nonwovens, for example the pore size and the mechanical strength, especially tensile strength. This post-processing refers to a chemical crosslinking and thus to the formation of covalent bonds between the molecules. A chemical crosslinking of molecule chains as well as individual nonwoven fibres at their contact points is particularly successful when using, for example, diisocyanates or diacrylates as suggested herein.

Especially by post-treatment of electrospun nanofibre nonwovens by plasma deposition processes (PECVD / *Plasma Enhanced Chemical Vapour Deposition*) ultra-thin layers can be generated while preserving the structure of the nonwoven fibres. Depending on the choice of starting materials for PECVD, these precursor monomers can change the mechanical properties, cause a change in surface polarity, or act as a permanent or biodegradable diffusion barrier, which ultimately has an effect on drug release and degradation processes.

As already mentioned, the crosslinking of the fibres with each other to form a crosslinked nonwoven material can be carried out by thermal post-treatment or by irradiating reactive light, especially UV light.

Further advantages are the modification of the surface morphology and the fibre diameters while preserving or modifying the mechanical properties, the guarantee of the shelf life of the materials with incorporated reactive molecules to allow processing in several process steps, and the use of nonwovens with reactive species embedded in the fibres for seamless joining of layered nonwoven materials.

By introducing reactive chemical substances such as diisocyanates or diacrylates, polymers can be adjusted covalently, by forming allophanate groups for example, or physically, by generating interpenetrating networks, in respect of their mechanical properties as well as with regard to their behaviour in the medium and their tendency to delamination. In addition, tear-resistant joins can be produced. Due to the given shelf life of the electrospun nanofibre nonwovens with incorporated reactive species, a further downstream processing of the materials is possible while preserving the intrinsic reactivity. The processes therefore can be embedded in a staggered process chain.

It should be emphasised that the characteristic fibre structure of the nanofibre nonwoven is retained in the post-process modifications described in accordance with the invention. By means of the methods described, adaptively electrospun nonwoven materials can be adjusted with regard to their physico-chemical properties, such as mechanics, surface polarity and finish, while preserving the same fibre structure. However, according to the invention, it is also possible to post-process the nonwoven morphology, such as surface structure and packing density, while maintaining the same mechanical properties.

In one embodiment, it is provided that a surface modification is applied to the fibres by applying at least one thin nano-layer, preferably in a thickness in the range of 400 to 600 nm.

By applying ultra-thin nano-layers (smaller than 2 µm, preferably smaller than 1 µm), for example by PECVD, it is also possible to create barrier layers which affect the interactions of the material with ambient media or to form diffusion barriers through these layers, which can ultimately have an effect on drug release processes and the degradation behaviour of the polymer materials.

Furthermore, the technology according to the invention opens up the possibility of applying polymeric layers by vapour deposition processes or ultra-thin polymeric layers by PECVD, which can fix the fibres while preserving the structure and, depending on the choice of the applied layer, may control the surface polarity or diffusion. The biological reaction as well as the degradation behaviour and the release rate of active substances incorporated in the nonwoven thus can be modulated.

The possibility of post-process modification of electrospun nanofibre nonwovens opens up a broad field of biomedical applications, among other things because the physico-chemical properties of a nonwoven material can be subsequently improved while preserving its morphology and can be adapted flexibly and, in part, spatially resolved to suit specific requirements.

Therefore, one aspect of the present invention is to use the nonwoven materials proposed herein as a component for medical devices, in particular medical implants. Accordingly, a further aspect of the present invention aims to provide a medical implant comprising a non-woven material proposed herein.

Such a medical implant can be a stent, for example in the form of a "covered" stent (stent graft), or a heart valve prosthesis and is particularly suitable for cardiovascular applications.

**In** the following, embodiments as well as further features and advantages of the invention will be explained by means of the figures, in which:

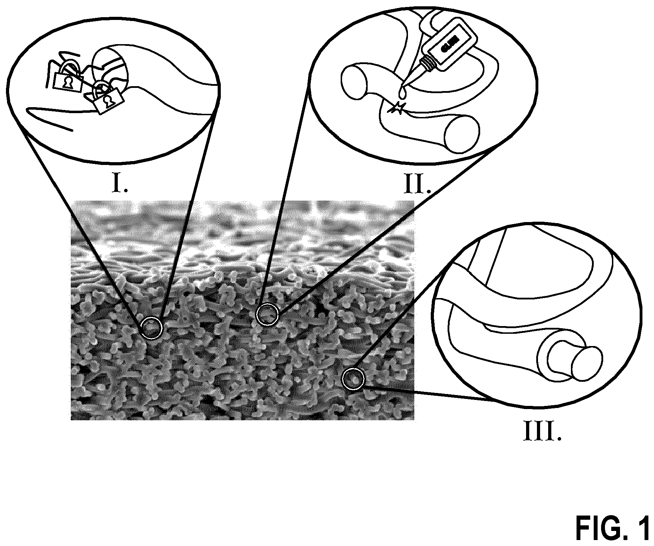

- Fig. 1: shows a schematic representation of the described post-processing of electrospun (nanofibre) nonwovens; and
- Fig. 2: shows a schematic representation of the post-process crosslinking of TSPCU with diisocyanates (A) and an overview of two interpenetrating polymer networks (B); and

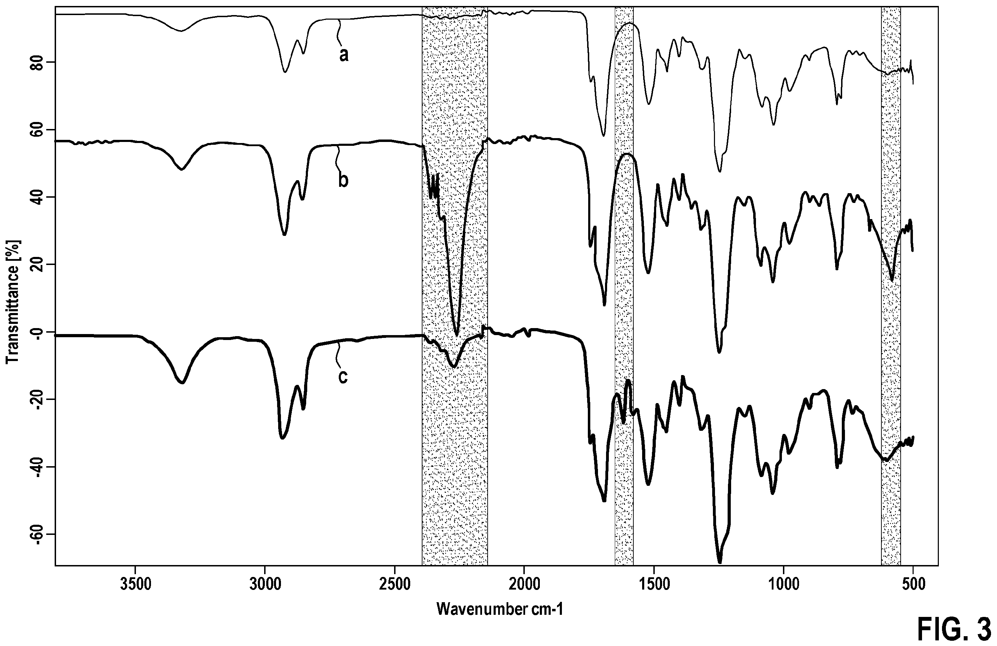

- Fig. 3: shows representative ATR-IR images of a TSPCU nonwoven as reference material (a) and of TSPCU nonwovens crosslinked with HMDI before thermal post-treatment (b) and after thermal post-treatment (c); and

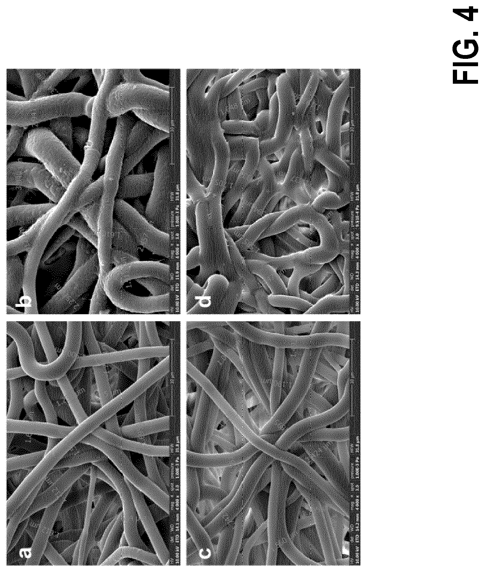

- Fig. 4: shows scanning electron microscope images of diisocyanate-crosslinked TSPCU nonwovens ((a) HMDI; (b) 4,4'-MBI, (c) Me3-HMDI and (d) 4,4'-DMDI), and

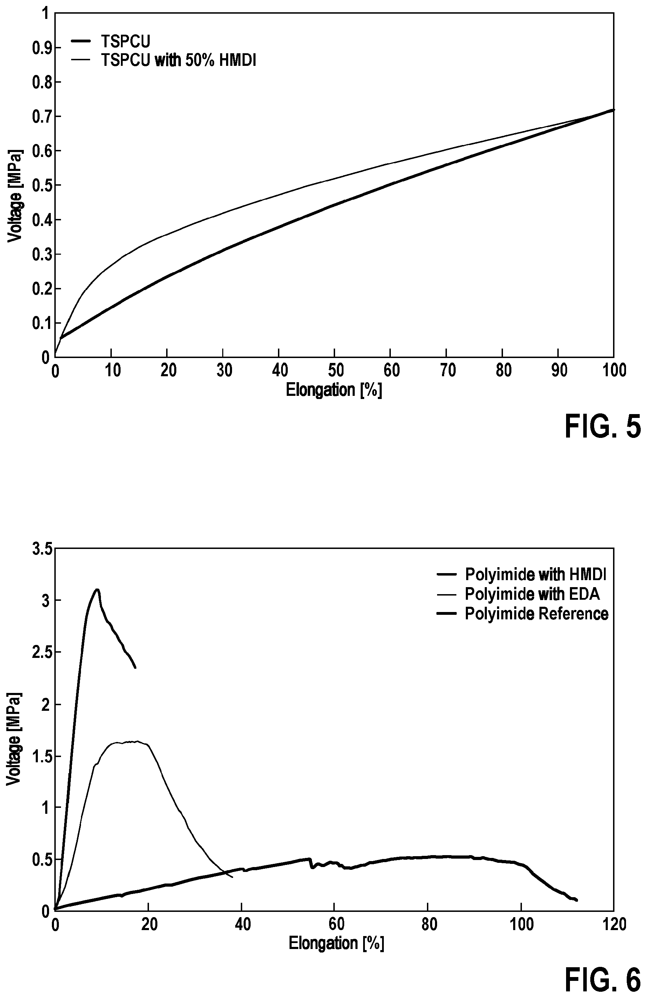

- Fig. 5: shows an exemplary stress-strain curve of hexamethylene-1,6-diisocyanate-crosslinked TSPCU nonwovens (light, top) compared to unmodified electrospun TSPCU (black, bottom); and
- Fig. 6: shows a further stress-strain curve of polyimide nonwovens spun with hexamethylene-1,6-diisocyanate (top) and ethylenediamine (middle) and then thermally treated compared to unmodified electrospun polyimide nonwovens (black, bottom); and

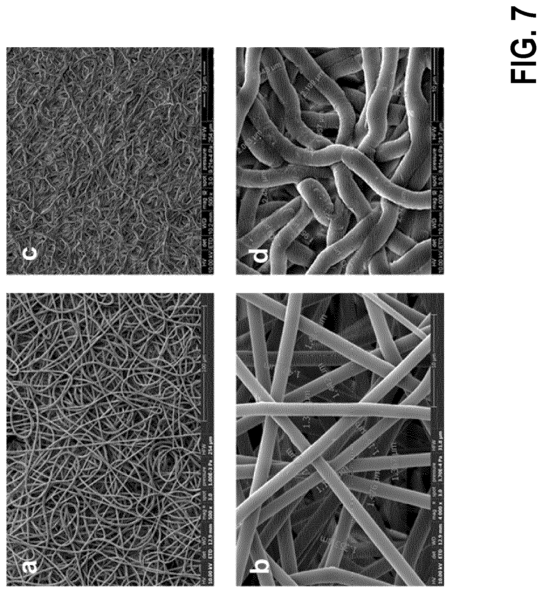

- Fig. 7: shows scanning electron microscope images of untreated electrospun TSPCU nanofibre nonwovens (a, b) compared with TSPCU nonwovens crosslinked by vapour deposition with HMDI (c, d); and

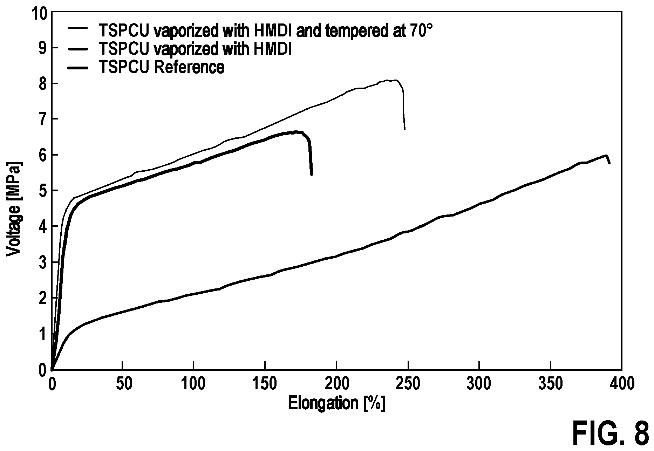

- Fig. 8: shows a stress-strain curve of untreated electrospun TSPCU compared to a TSPCU nonwoven crosslinked by vapour deposition with HMDI; and

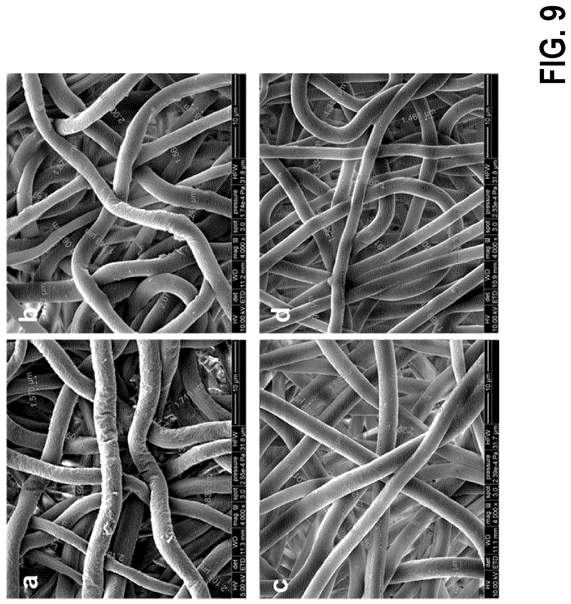

- Fig. 9: shows scanning electron microscope images of electrospun TSPCU nanofibre nonwovens coated with polyallylamine under variation of the process parameters ((a) parameters: 1 min, 1.20 mbar, 60% input (i.e. initial power) and (b) parameters: 5 min, 0.90 mbar, 60% input (i.e. initial power)) and HMDSO ((c) parameters: 10 min, 0.59 mbar, 10% input (i.e. initial power), (d) parameters: 5 min, 0.77 mbar, 10% input (i.e. initial power))
- Fig. 10: shows the relative stoichiometric composition of TSPCU nonwovens after plasma coating with allylamine (parameters: 1 min, 1.20 mbar, 60% input (i.e. initial power)), and allylamine (parameters: 5 min, 0.90 mbar, 60% input (i.e. initial power)), and HMDSO (parameters: 10 min, 0.59 mbar, 10% input (i.e. initial power)) and HMDSO (parameters: 5 min, 0.77 mbar, 10% input (i.e. initial power)); and

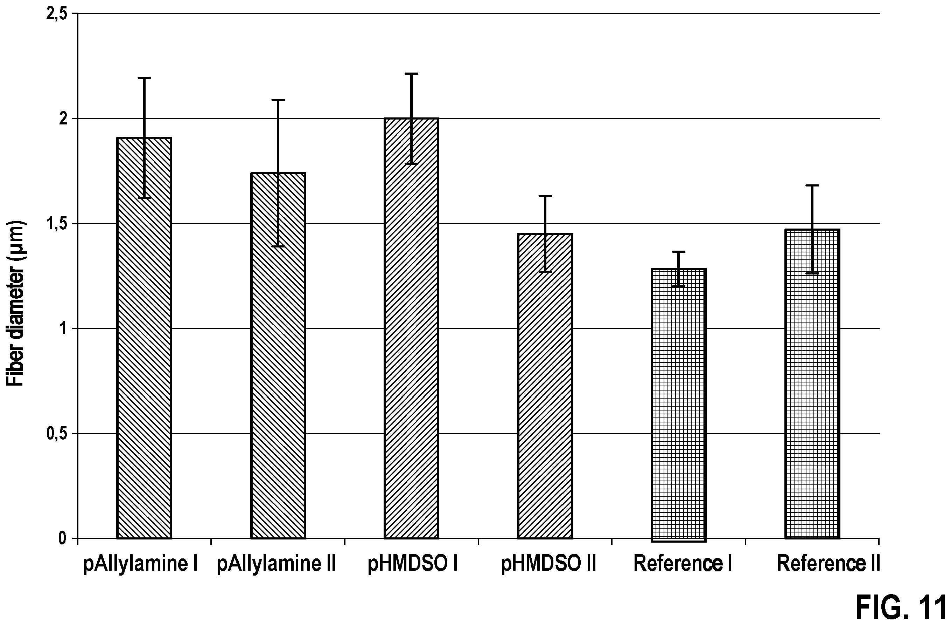

- Fig. 11: shows the development of the fibre diameters of TSPCU nonwovens after plasma coating with allylamine (parameters: 1 min, 1.20 mbar, 60% input (i.e. initial power)) and allylamine (parameters: 5 min, 0.90 mbar, 60% input (i.e. initial power)), as well as HMDSO (parameters: 10 min, 0.59 mbar, 10% input (i.e. initial power)) and HMDSO (parameters: 5 min, 0.77 mbar, 10% input (i.e. initial power)) in comparison to an uncoated TSPCU nonwoven; and

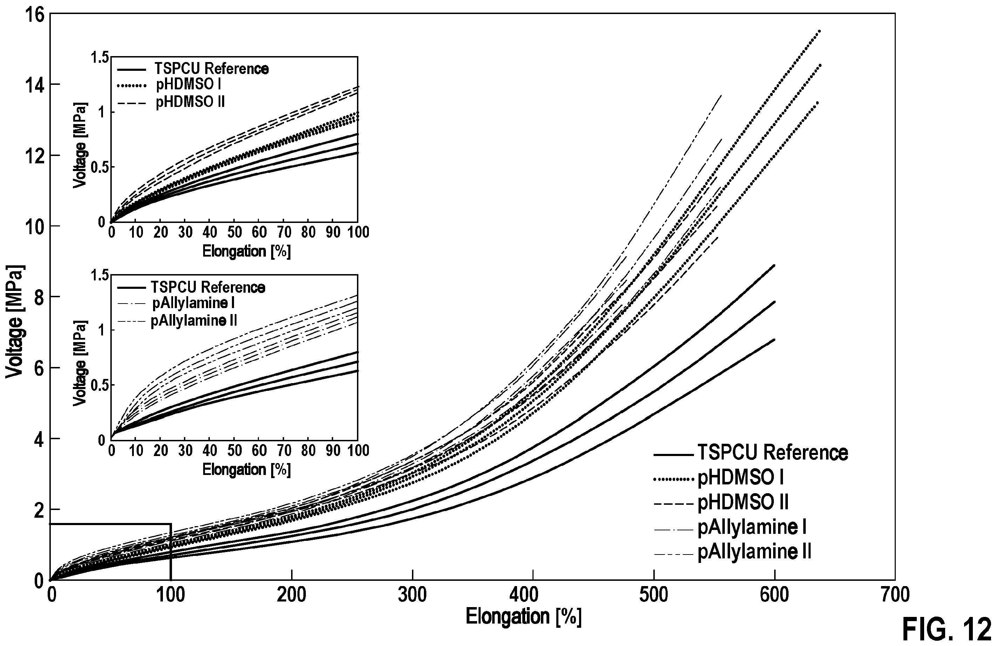

- Fig. 12: shows mean stress-strain curves of TSPCU nonwovens after plasma coating with allylamine (parameters: 1 min, 1.20 mbar, 60% input (i.e. initial power)) and allylamine (parameters: 5 min, 0.90 mbar, 60% input (i.e. initial power)) and HMDSO (parameters: 10 min, 0.59 mbar, 10% input (i.e. initial power)) and HMDSO (parameters: 5 min, 0.77 mbar, 10% input (i.e. initial power)) in comparison to an uncoated TSPCU nonwoven (the middle line of each depicted type of lines is the mean and the respective lines of same type surrounding each central mean line correspond to the standard deviation (n = 6)).

### Practical examples

### Example 1

The polymer solution used for electrospinning, for example 7.5 wt.% thermoplastic silicone polycarbonate-urethane (TSPCU) in chloroform (CHCl₃), trifluoroethanol (TFE) and dimethylformamide (DMF), is supplemented by reactive components added in a ratio of 1 to 50% relative to the polymer mass, and the mixture is spun within 6 hours. These reactive components include diisocyanates, such as hexamethylene diisocyanate (HMDI), methylene-(bisphenyl-isocyanate) or lysine diisocyanate. The functional groups of the isocyanates are retained during the spinning process and are homogeneously distributed in the polymer fibres. The reaction of the isocyanates with the provided functional groups of the polymer, such as urethane, amide, amine or alcohol groups, is stimulated by thermal treatment of the nonwoven thus obtained, and a chemical crosslinking process takes place both within the fibres and at the contact points of individual fibres, so that the fibres are covalently bonded to each other and can no longer be displaced relative to each other. This results in a reduced tendency to delamination and increased tensile strength of the nonwoven compared to the untreated material.

In particular, it has been shown that nonwovens with incorporated diisocyanates obtained in this way can be stored at -20°C without losing the reactive components. This is interesting in that a processing with a number of individual process steps can be added. This technology can be used to embed structures, for example a stent, into the nonwoven scaffold without risking a separation of the fibres at the insertion point. This allows seamless j oining, which results in a much higher tearing strength.

The incorporation of reactive components into the spinning process can also be extended to other electrospun polymers and is not limited to TSPCU. For example, the incorporation of HMDI or allylamine during the spinning process of polyimide with subsequent thermal post-treatment results in a strong increase of the tensile strength or stabilisation of the nonwoven compared to delamination.

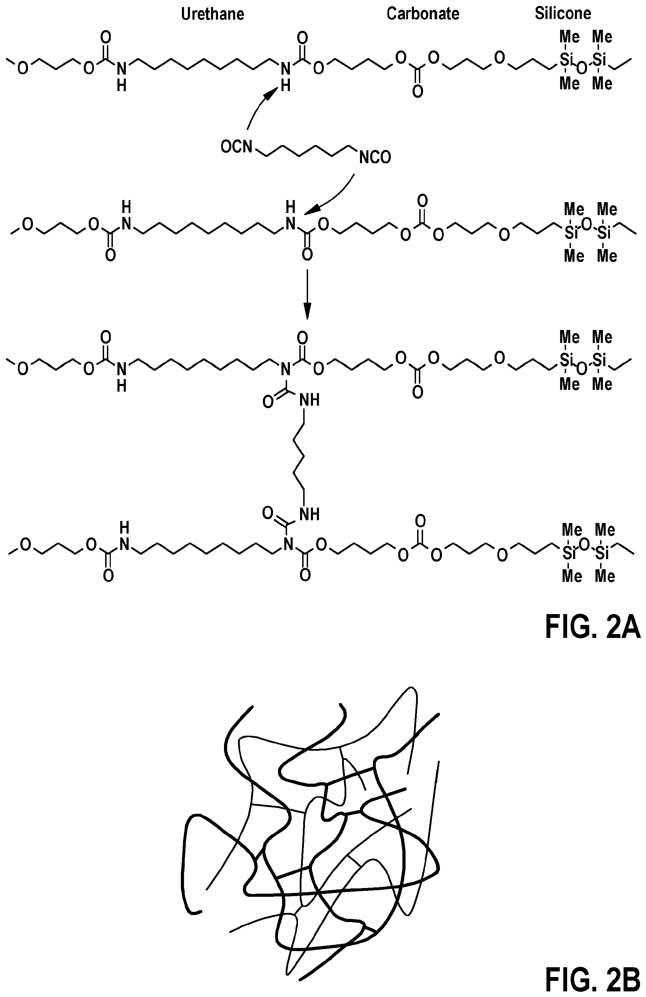

In addition, a purely physical crosslinking of the individual polymer chains can also take place in the absence of sufficient reactive groups, by forming an interpenetrating network (see Fig. 2 II), based on the diisocyanates. A further possibility of forming such an interpenetrating network is the use of diacrylates in the spinning process and subsequent photoinduced crosslinking of these acrylates. In this way, the polymer chains of the electrospun material are intertwined with oligoacrylates. According to the invention, the macroscopic structure of the nonwoven is maintained, with a simultaneous change in the mechanical stability of the material.

### Example 2

By vapour deposition of electrospun nanofibre nonwovens by reactive species such as volatile diisocyanates, layers can be applied to the individual fibres, which on the one hand fix them against displacement of the nonwoven fibres among themselves and on the other hand can shield them against solvent effects, such as hydrating of hydrogen bonds. For post-processing, the nonwoven material is incubated at room temperature and 70°C in a saturated steam atmosphere of diisocyanate to achieve surface crosslinking. The fibre structure of the material is preserved by the gentle vaporisation process.

Depending on the choice of vaporising materials, as well as the temperature and duration of the process, the thickness of the layer applied to the nonwoven can be modified according to the requirements. In addition, the effect of delamination of electrospun nonwovens can be minimised by this process, as the fibres crosslink with each other on a macroscopic level. By means of the described post-processing, materials are obtained which have a modified fibre morphology and fibre surface as well as increased packing density of the fibres while preserving the mechanical properties.

### Example 3

A post-process application of ultra-thin layers in the range of 1 - 2 µm to the fibres of the nanofibre nonwoven by *Plasma Enhanced Chemical Vapour Deposition* (PECVD) is also described in accordance with the invention. Here, monomers excited by low-pressure plasma are made to polymerise on the nonwoven surface and form polymer layers on the fibres while preserving the fibre morphology. This process is particularly impressive because of the wide range of monomers that can be used for the application process. This makes it possible to fix the nonwoven fibres against each other in a similar way to a vaporisation process or to shield them from the surrounding medium by the applied layer. The low thickness of the applied layers is remarkable. Furthermore, by using degradable starting materials, e.g. acrylate-functionalised esters, a degradable layer can be applied to the individual fibres, which modifies degradation and release processes. Depending on the choice of the monomer used, these layers also have a high ductility, which is particularly positive in respect of their use as a coating technology for strongly moving parts, such as those found in a TAVI or stent. By selecting the process parameters of the PECVD, the applied coatings can be modified in their stoichiometry as well as their structural composition, such as coating thickness. The described technique of applying ultra-thin layers by PECVD can be used for both biostable and biodegradable polymers and is not limited to certain classes of polymers.

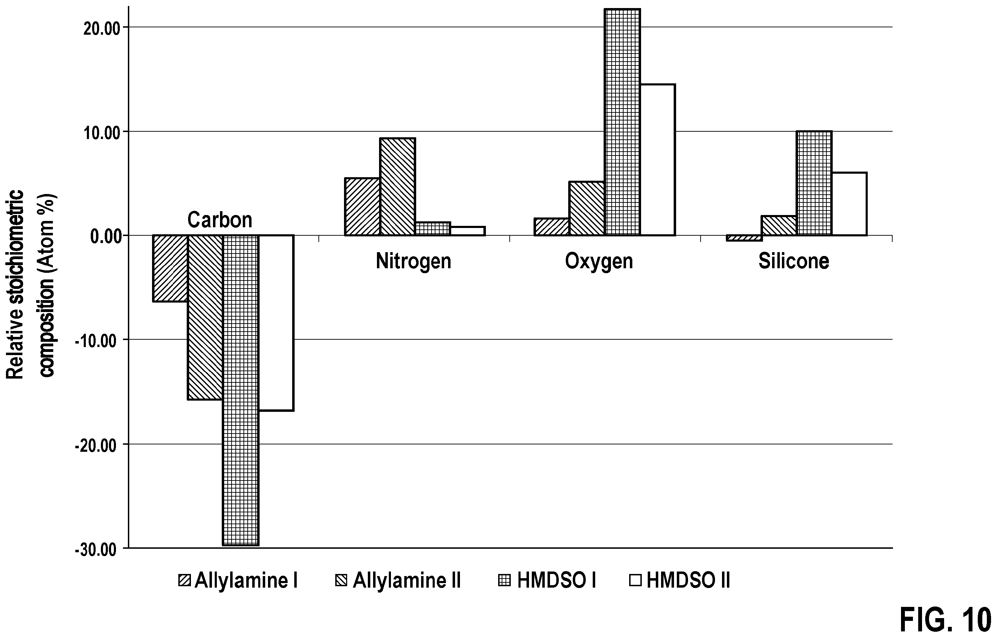

The change in the stoichiometry when applying ultra-thin layers of p-allylamine -and p-hexamethyldisiloxane (HDMSO) compared to untreated TSPCU is shown in Fig. 10 as a differential representation. The percentage decrease in carbon can be seen with a simultaneous increase in the nitrogen or silicon content.

## Claims

1. A medical implant comprising a nonwoven material formed from electrospun fibres having a surface modification by which the fibres can be crosslinked together.

2. The medical implant according to claim 1, **characterized in that** the fibres are crosslinked.

3. The medical implant according to claim 1 or 2, **characterized in that** the surface modification is in the form of chemical reactive groups.

4. The medical implant according to claim 3, **characterized in that** the chemical reactive groups are selected from the group comprising or consisting of diisocyanates, alcohols, epoxides, imides, amides, imines, amines, in particular allylamines, diacrylates, disiloxanes and disilazanes.

5. The medical implant according to any of the preceding claims, **characterized in that** the fibrous material is selected from the group comprising or consisting of polyurethanes, polyimides, polyamides, polyesters and polytetrafluoroethylenes.

6. The medical implant according to claim 5, **characterized in that** the fibre material is thermoplastic silicone polycarbonate-urethane.

7. A method for producing a medical implant comprising a crosslinked electrospun nonwoven material comprising the steps of:
- providing a fibre material,
- electrospinning the fibre material in the form of fibres into a nonwoven material,
- introducing a surface modification to the fibres by modifying the fibre material before the electrospinning,
- crosslinking the fibres to form a crosslinked nonwoven material.

8. The method according to claim 7, **characterized in that** the introduction of a surface modification is carried out by adding a reactive chemical substance to the fibre material before the electrospinning.

## Patentansprüche

1. Medizinisches Implantat, das ein Vliesmaterial umfasst, das aus elektrogesponnenen Fasern mit einer Oberflächenmodifikation, durch die die Fasern miteinander vernetzt werden können, gebildet ist.

2. Medizinisches Implantat nach Anspruch 1, **dadurch gekennzeichnet, dass** die Fasern vernetzt sind.

3. Medizinisches Implantat nach Anspruch 1 oder 2, **dadurch gekennzeichnet, dass** die Oberflächenmodifikation in Form von chemisch reaktiven Gruppen besteht.

4. Medizinisches Implantat nach Anspruch 3, **dadurch gekennzeichnet, dass** die chemisch reaktiven Gruppen ausgewählt sind aus der Gruppe, die Diisocyanate, Alkohole, Epoxide, Imide, Amide, Imine, Amine, insbesondere Allylamine, Diacrylate, Disiloxane und Disilazane umfasst oder daraus besteht.

5. Medizinisches Implantat nach einem der vorhergehenden Ansprüche, **dadurch gekennzeichnet, dass** das Fasermaterial aus der Gruppe ausgewählt ist, die Polyurethane, Polyimide, Polyamide, Polyester und Polytetrafluorethylene umfasst oder daraus besteht.

6. Medizinisches Implantat nach Anspruch 5, **dadurch gekennzeichnet, dass** das Fasermaterial ein thermoplastisches Silikon-Polycarbonat-Urethan ist.

7. Verfahren zur Herstellung eines medizinischen Implantats, das ein vernetztes elektrogesponnenes Vliesmaterial umfasst, die folgenden Schritte umfassend:
- Bereitstellung eines Fasermaterials,
- Elektrospinnen des Fasermaterials in Form von Fasern zu einem Vliesstoff,
- Einführung einer Oberflächenmodifikation der Fasern durch Modifizierung des Fasermaterials vor dem Elektrospinnen,
- Vernetzung der Fasern zur Bildung eines vernetzten Vliesstoffs.

8. Verfahren nach Anspruch 7, **dadurch gekennzeichnet, dass** die Einführung einer Oberflächenmodifikation durch Zugabe einer reaktiven chemischen Substanz zu dem Fasermaterial vor dem Elektrospinnen erfolgt.

## Revendications

1. Implant médical comprenant un matériau non tissé formé de fibres électrofilées présentant une modification de surface par l'intermédiaire de laquelle les fibres peuvent être réticulées ensemble.

2. Implant médical selon la revendication 1, **caractérisé en ce que** les fibres sont réticulées.

3. Implant médical selon la revendication 1 ou 2, **caractérisé en ce que** la modification de surface est sous la forme de groupes chimiques réactifs.

4. Implant médical selon la revendication 3, **caractérisé en ce que** les groupes chimiques réactifs sont choisis dans le groupe comprenant ou consistant en des diisocyanates, alcools, époxydes, imides, amides, imines, amines, en particulier allylamines, diacrylates, disiloxanes et disilazanes.

5. Implant médical selon l'une quelconque des revendications précédentes, **caractérisé en ce que** le matériau fibreux est choisi dans le groupe comprenant ou consistant en des polyuréthanes, polyimides, polyamides, polyesters et polytétrafluoroéthylènes.

6. Implant médical selon la revendication 5, **caractérisé en ce que** le matériau fibreux est du silicone-polycarbonate-uréthane thermoplastique.

7. Procédé de production d'un implant médical comprenant un matériau non tissé électrofilé réticulé, comprenant les étapes consistant à :
- fournir un matériau fibreux,
- électrofiler le matériau fibreux sous la forme de fibres en un matériau non tissé,
- apporter une modification de surface aux fibres en modifiant le matériau fibreux avant l'électrofilage,
- réticuler les fibres pour former un matériau non-tissé réticulé.

8. Procédé selon la revendication 7, **caractérisé en ce que** l'introduction d'une modification de surface est effectuée en ajoutant une substance chimique réactive au matériau fibreux avant l'électrofilage.
